# EUROPEAN PATENT APPLICATION

(11) **EP 3 214 184 A1**
(43) Date of publication of application: **06.09.2017**
(21) Application number: 17157200.1
(22) Date of filing: 08.09.2009
(51) Int. Cl.: C12Q 1/68, G01N 33/68

(54) **METHODS FOR DIAGNOSING CANCER AND DETERMINING THE OVERALL SURVIVAL AND DISEASE-FREE SURVIVAL OF CANCER PATIENTS**

(30) Priority: 05.09.2008 US 94726 P; 12.12.2008 US 122130 P
(62) Divisional of application: 09812382.1
(71) Applicant: A & G Pharmaceutical, Inc., Columbia, MD 21045 (US)
(72) Inventor: Serrero, Ginette, Columbia, MD 21045 (US)
(74) Representative: Potter Clarkson LLP

(57) **Abstract**

The invention provides methods for prognosis of patients afflicted with cancer, comprising determining the level of GP88 expression in a biological sample obtained from said patient.

## Description

### BACKGROUND OF THE INVENTION

### Field of Invention

The invention generally relates to diagnostic and monitoring methods and assays for cancer and kits that may be used in such methods. More particularly, the application relates to the use of GP88 expression for predicting the likelihood of metastasis, length of disease-free survival and length of overall survival of cancer patients and the outcome of cancer therapies.

### Related Art

Breast cancer is the leading form of cancer in women, and the second leading cause after lung cancer of cancer deaths in the United States. In the industrialized world, about one woman in every nine can expect to develop breast cancer in her lifetime. In the United States, the annual incidence of breast cancer is about 180,000 new cases and approximately 48,000 deaths each year (Parkin 1998; Apantaku 2000). Approximately two million women living in the United States alone have been diagnosed with breast cancer at some point in their lives. Despite ongoing improvements in understanding the disease, breast cancer has remained to a large extent resistant to medical intervention. Most clinical initiatives are focused on early diagnosis, followed by conventional forms of intervention, particularly surgery, radiation, hormone suppression, and chemotherapy. Such interventions are of limited success, particularly in patients where the tumor has undergone metastasis. There is a pressing need to improve the arsenal of diagnostic tools and methods available to provide more precise and more effective information that will allow successful treatment in the least invasive way possible. Specifically, markers that can identify patients with very low risk of disease reappearance, metastasis, and death after initial surgery would reduce the extent of over treatment with expensive and potentially toxic supplementary regimes. The invention meets that need by providing new methods and markers for monitoring breast cancer.

Among the large group of breast cancer patients with localized tumors and without detectable metastases to nearby lymph nodes, many will be cured by surgery because the tumors have not metastasized to surrounding tissues and lymph nodes. However, others have occult metastatic disease and could benefit from supplementary radiation or adjuvant anti-hormone therapy or chemotherapy. There is a need for diagnostic markers to discriminate between tumors with low risk for metastatic metastasis and those with higher risk. Tumor markers that signify low risk of metastatic disease may directly affect the therapeutic decision of whether to use supplementary radiation or adjuvant hormone or chemotherapy. Furthermore, such tumor markers may also affect the surgeon's recommendation of whether to choose breast conserving surgery or mastectomy.

### Diagnosis of Breast Cancer

The definitive diagnosis of all types of breast disease is based on histologic evaluation of tissue samples using the light microscope. The histologic criteria used to define most breast lesions are historic but nonetheless quite reproducible for identifying fully invasive breast cancers. Recent accomplishments include the identification of a small number of tissue-based biomarkers that are helpful in predicting clinical outcome and response to therapy (e.g., S-phase fraction, estrogen and progesterone receptors, c-erbB-2) and the discovery of genes (BRCA-1 and BRCA-2) associated with familial risk for breast cancers (Dahiya and Deng 1998; Fitzgibbons, Page et al. 2000).

The molecular basis of cancer is still being determined. In breast cancer, receptors for estrogen and progesterone are related to the state of mammary epithelial cell differentiation and have prognostic value for disease outcome in certain cases. Estrogen is known to be a primary stimulator for estrogen receptor (ER) positive human breast cancer cell growth in vivo and in vitro. Although estrogen is initially required for establishment and proliferation of breast tumors, the development of estrogen-independent tumors during the course of breast cancer is indicative of poor prognosis. It has been postulated that the mitogenic effect of estrogen in breast cancer cells is mediated, at least partially, by autocrine growth factors, including growth factors regulated by estrogen. Thus, the identification and characterization of estrogen-responsive genes, particularly genes encoding growth factors, contributes to the understanding of the effects of estrogen in breast cancer cells.

However, diagnosing breast cancer still requires some type of biopsy procedure. In addition, current diagnostic and prognostic methods cannot absolutely distinguish breast cancers that are treatable by surgery alone from those that are likely to reappear or have already metastasis through metastases. As a result, at least 50 percent of breast cancer patients are treated with some form of adjuvant therapy. Moreover, available methods are inadequate for predicting the response of breast cancers to specific types of adjuvant therapies.

Treatment decisions for individuals afflicted with breast cancer are frequently based on the number of axillary lymph nodes involved with disease, estrogen receptor and progesterone receptor (PR) status, size of the primary tumor, and stage of disease at diagnosis (Tandon, Clark et al. 1989). However, even with this variety of factors, it is currently not possible to predict accurately the course of disease. There is clearly a need to identify new markers in order to separate patients with good prognosis, who might need no supplementary therapy beyond surgical removal of the malignant breast tumor, from those whose cancer is more likely to reappear and who might benefit from additional and more exhaustive treatment forms.

There remain deficiencies in the art with respect to the identification of markers linked with the progression of breast cancer, the development of diagnostic methods to monitor disease progression and the development of therapeutic methods and compositions to treat breast diseases and cancers. The identification of markers which are differentially expressed or activated in breast cancer would be of considerable importance in the development of a rapid, inexpensive method to improve diagnosing of breast cancer and to predict tumor behavior with respect to patient prognosis and responsiveness to individual therapeutic options.

### GP88

The 88 kDa glycoprotein PC cell-derived growth factor (PCDGF) is an autocrine growth factor, first isolated from the elevatedly tumorigenic mouse teratoma PC cells. PCDGF also known in the art as GP88, Granulin-Epithelin Precursor (GEP), Granulin Precursor, Progranulin, Epithelin Precursor, Proepithelin (PEPI) and Acrogranin (herein after referred to as GP88), is the largest member of the granulin/epithelin family of cysteine-rich polypeptide growth modulators. It has been reported that GP88 stimulated the proliferation and survival of several cell types of mesenchymal and epithelial origin by stimulating MAP kinase, PI-3 kinase and FAK kinase pathways. Interestingly, over-expression of GP88 was found in several cancer cell lines and/or tumor tissues including breast cancer, ovarian cancer, renal cell carcinoma, multiple myeloma and glioblastoma.

In breast cancer cells, GP88 has been shown to play a critical role in tumorigenesis. GP88 over-expression correlated positively with the acquisition of estrogen-independent growth, tamoxifen resistance and tumorigenicity. Inhibition of GP88 expression by antisense cDNA transfection in MDA-MB-468 cells resulted in a complete inhibition of tumor growth in nude mice. In addition, it was demonstrated that GP88 prevented the apoptotic effect of tamoxifen in estrogen receptor positive breast cancer cells. Pathological studies with breast carcinoma biopsies revealed that GP88 was expressed in 80% invasive ductal carcinoma in correlation with poor prognosis markers such as tumor grade, p53 expression and Ki67 index whereas benign lesions were mostly negative. In addition, pathological studies in ovarian tumors indicated that GP88 was elevatedly expressed in invasive epithelial ovarian tumors when compared with tumors with low malignant potential. These studies demonstrate that GP88 plays a role in invasion in addition to stimulating proliferation of tumor cells.

Granulins/epithelins ("grn/epi") are 6 kDa polypeptides and belong to a novel family of double cysteine rich polypeptides. U.S. Pat. No. 5,416,192 (Shoyab et al.) is directed to 6 kDa epithelins, particularly epithelin 1 and epithelin 2. According to Shoyab, both epithelins are encoded by a common 63.5 kDa precursor, which is processed into smaller forms as soon as it is synthesized, so that the only natural products found in biological samples are the 6 kDa forms. GP88 is this epithelin precursor, and Shoyab et al. teach that GP88 is biologically inactive.

Contrary to the teachings of Shoyab et al., the present inventor's laboratory has demonstrated that the precursor is not always processed as soon as it is synthesized. Studies have demonstrated that the precursor (i.e., GP88) is in fact secreted as an 88 kDa glycoprotein with an N-linked carbohydrate moiety of 20 kDa. Analysis of the N-terminal sequence of GP88 indicates that GP88 starts at amino acid 17 of the grn/epi precursor, demonstrating that the first 17 amino acids from the protein sequence deduced from the precursor cDNA correspond to a signal peptide compatible with targeting for membrane localization or for secretion. In contrast to the teachings of Shoyab et al., GP88 is biologically active and has growth promoting activity, particularly as an autocrine growth factor for the producer cells.

There remains a need in the art for markers linked with the progression of cancers (e.g., breast and lung cancers) and diagnostic methods for predicting disease progression based on such markers. These needs and others are met by the present invention.

### SUMMARY OF THE INVENTION

It has been discovered by the inventors that elevated levels of GP88 expression is associated with increased likelihood of metastasis, decreased overall survival, and decreased disease-free survival of patients afflicted with cancer (e.g., breast and lung cancer). This association was particularly strong for estrogen receptor negative (ER-), estrogen receptor positive and lymph node negative (ER+/LN-), and estrogen receptor positive and lymph node positive (ER+/LN+) breast cancers. Such an association was not known prior to the inventors' discovery. The inventors show that the presence of elevated GP88 expression in human breast cancer (particularly, in invasive ductal carcinoma) is associated with an increased likelihood of metastasis, a decreased disease-free and overall survival, and therefore, GP88 expression is a new and novel prognostic marker of breast cancer progression. Elevated levels of GP88 expression in the primary tumor(s) of patients is a strong positive prognostic factor.

The inventors show that the presence of elevated GP88 expression in human lung cancer (particularly, in non small cell lung carcinoma (NSCLC)) is associated with an increased likelihood of metastasis, a decreased disease-free and overall survival, and therefore, GP88 expression is a new and novel prognostic marker of lung cancer progression. Elevated levels of GP88 expression in the primary tumor(s) of patients is a strong positive prognostic factor.

Levels of GP88 expression may be analyzed using any technique known to those in the art, for example, with antibodies or other compositions capable of binding GP88 (e.g., ligands, etc.,). Thus, the analysis of GP88 expression adds a new level of information to current cancer (e.g., breast and lung cancer) markers and is a reliable prognostic molecular/biochemical marker of cancer in samples from patients afflicted with cancer. Additionally, monitoring levels of GP88 expression is predictive of the outcome of effective therapeutic strategies for breast cancer patients.

In accordance with the present invention, methods are provided for prognosis of length of disease-free or overall survival in a patient suffering from cancer. In one embodiment, it has been found that elevated levels of GP88 expression show an unexpected and surprisingly elevated correlation to decreased length of disease-free and/or overall survival.

Thus, the present invention advantageously provides a significant advancement in cancer management because early identification of patients at risk for tumor reappearance or metastasis will permit aggressive early treatment with significantly enhanced potential for survival.

Alternatively, the instant invention provides methods for predicting the length of disease-free and overall survival; predicting progression-free survival, predicting event-free survival, predicting the risk of decreased disease-free or overall survival, predicting the likelihood of recovery of a patient suffering from cancer; predicting the likelihood of reappearance of cancer and/or metastasis in an individual having a cancer tumor; predicting the risk of reappearance of cancer, methods for screening a patient suffering from cancer to determine the risk of tumor metastasis; methods for determining the proper course of treatment for a patient suffering from cancer; methods for monitoring the effectiveness of a course of treatment for a patient suffering from cancer; and kits for use in practicing the invention methods.

### BRIEF DESCRIPTION OF DRAWINGS

FIG. 1 shows Kaplan-Meier curves that were generated for ER- patients after prognostic classification based on staining with anti-GP88 antibody.
Fig. 2 shows Kaplan-Meier curves that were generated for ER+/LN- patients after prognostic classification of disease-free survival based on staining with anti-GP88 antibody.
FIG.3 shows Kaplan-Meier curves that were generated for ER+/LN- patients after prognostic classification of overall survival based on staining with anti-GP88 antibody.
Fig. 4 shows Kaplan-Meier curves that were generated for ER+/LN+ patients after prognostic classification of disease-free survival based on staining with anti-GP88 antibody.
FIG. 5 shows Kaplan-Meier curves that were generated for ER+/LN+ patients after prognostic classification of overall survival based on staining with anti-GP88 antibody.
FIG. 6 shows Immunohistochemical detection of various levels of GP88 expression in normal and breast cancer tissues using an anti-GP88 antibody.
FIG. 7 is a graph showing the optical density (y-axis) of samples containing known quantities of GP88 (x-axis). The graph can be used as a reference to determine the concentration of GP88 in a biological fluid sample such as blood serum.
FIG. 8 shows circulating level of GP88 for breast cancer patients (BC Pts) that have no evidence of disease and that have progressive disease.
FIG. 9 shows the level of GP88 for breast cancer patients with early stage (stage 2) disease having no evidence of disease.
FIG. 10 shows the level of GP88 when patients that have early stage disease relapse to stage 4 (metastatic disease).
FIG. 11 shows that the maintenance of a elevated GP88 level for several weeks lead to a decrease in survival (A) Patient 1, and (B) Patient 2.

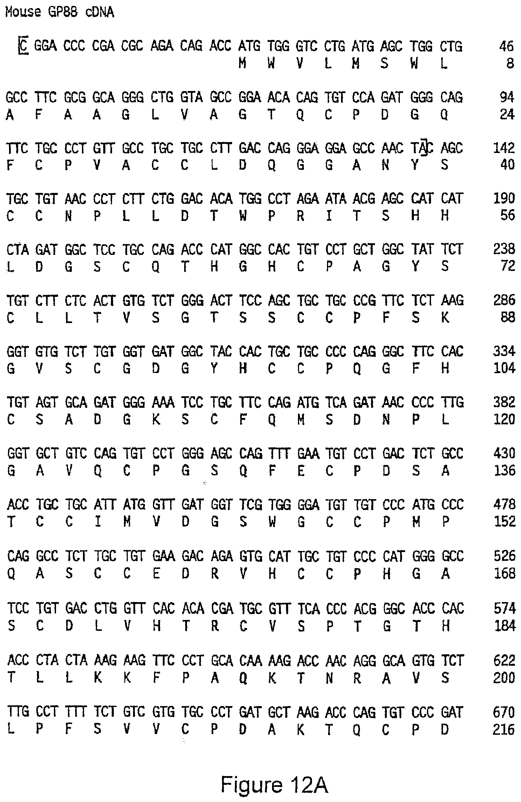

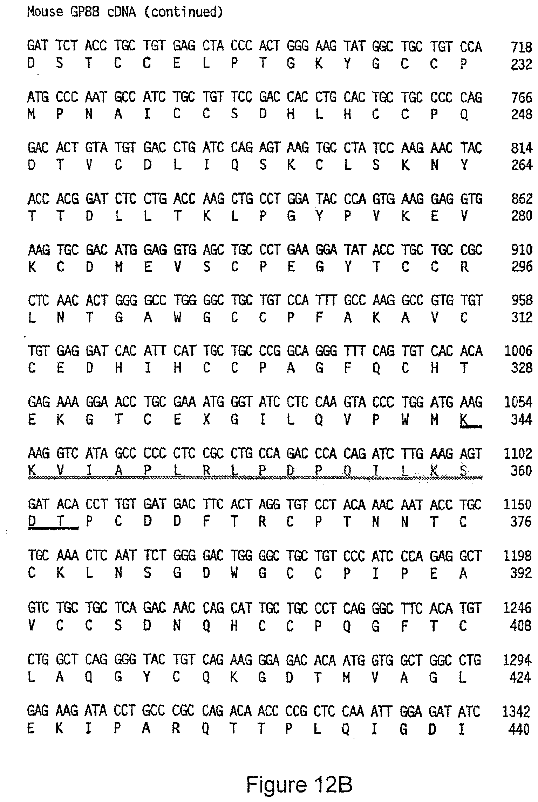

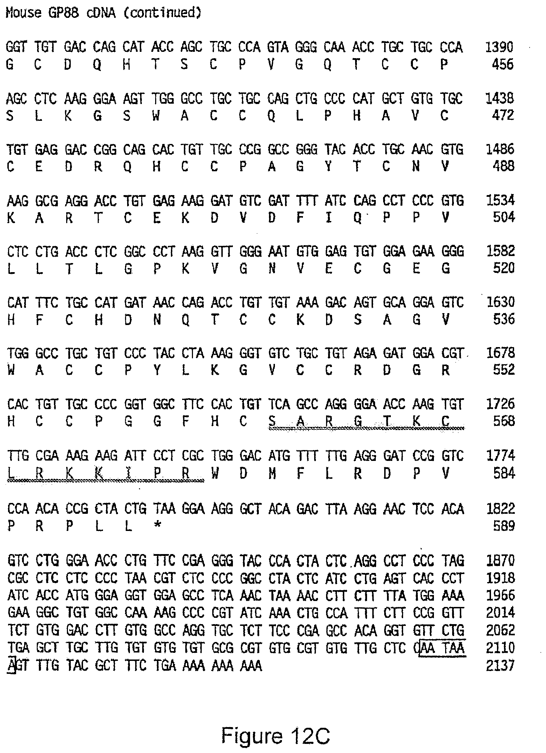

FIGS. 12A-C show the nucleotide and deduced amino-acid sequence of mouse GP88.

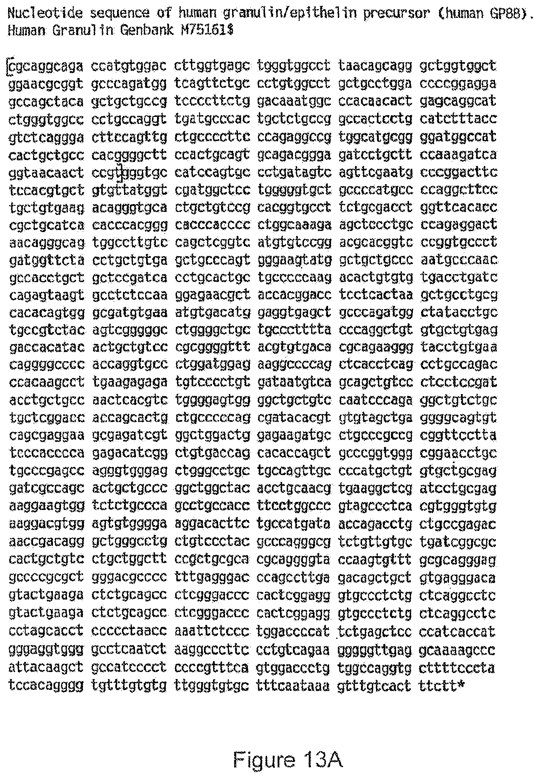

FIG. 13A shows the nucleotide sequence of human GP88 cDNA.

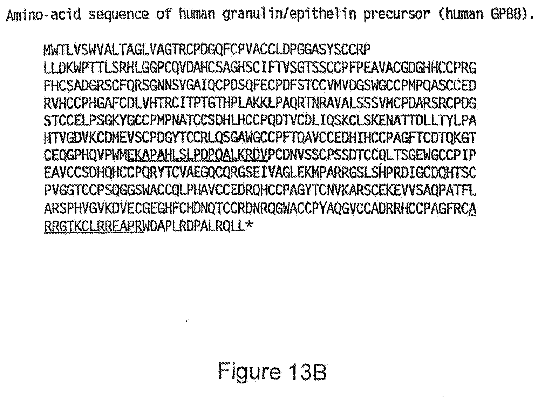

FIG. 13B shows the amino-acid sequence of human GP88.
FIG. 14 shows GP88 staining scores in Formalin-Fixed Paraffin-Embedded (FFPE) tissue from Non-small cell lung cancer (NSCLC) biopsy fitted for disease free survival (DFS) which reveals that increased GP88 expression correlates with decreased DFS. Biostat Analysis showed a strong connection between GP88 and recurrence (p=0.0091).
FIG. 15 shows GP88 staining scores in FFPE tissue from Stage 1 and Stage 2 NSCLC biopsy fitted for overall survival (OS). Biostat Analysis revealed a strong connection between GP88 and death (p=0.0038). Each one unit increase of GP88 is associated with a 65% to 83% increase risk of dying.

### DETAILED DESCRIPTION OF THE INVENTION

In accordance with the present invention, methods are provided for prognosis of a patient afflicted with cancer, comprising determining the levels of GP88 expression in a biological sample obtained from said patient.

In one embodiment, the method may comprise contacting said biological sample with a GP88 binding composition.

In another embodiment, the method may further comprise comparing the levels of GP88 expression in said biological sample to a standard, and thereby providing for prognosis associated with said determined levels of GP88 expression.

For example, in one embodiment of the invention, it has been discovered that elevated levels of GP88 expression is associated with patients having a decreased length of survival.

For example, in one embodiment of the invention, it has been discovered that elevated levels of GP88 expression is associated with patients having a decreased length of overall survival.

In another embodiment, it has been found that elevated levels of GP88 expression are associated with patients having a decreased length of disease-free survival.

In one embodiment of the invention, it has been discovered that elevated levels of GP88 expression is associated with patients having a decreased length of progression-free survival.

In another embodiment, it has been found that elevated levels of GP88 expression are associated with patients having a decreased length of event-free survival.

The levels of GP88 expression may be used as the sole factor in assessing the disease status, or along with the additional factors, including, in the illustrative case of breast cancer, lymph node status, estrogen receptor status, and the like.

The invention methods are useful in the prognosis of individuals with neoplastic diseases, including both solid tumors and hematopoietic cancers. Exemplary neoplastic diseases include carcinomas, such as adenocarcinomas and melanomas; and sarcomas, such as various leukemias or lymphomas. Of particular interest are cancers of breast, liver, kidney, testes, brain, ovary, skin, lung, prostate, thyroid, pancreas, cervix, colorectal, stomach, intestine, bladder, hematopoietic (lymphoid and myeloid), gastrointestinal (e.g., colon), genitourinary tract (e.g., renal, urothelial cells), uterine, head and neck and nasopharynx; particularly breast cancer, more particularly invasive ductal carcinoma. Still further examples of tumors include benign tumors including, but not limited to hemangiomas, acoustic neuromas, neurofibromas, trachomas, and pyogenic granulomas; other malignancies such as most rectal cancer, renal-cell carcinoma, non-small cell carcinoma of the lung, cancer of the small intestine and cancer of the esophagus. Still further examples of tumors include: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, gastrointestinal system carcinomas, colon carcinoma, genitourinary system carcinomas, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, endocrine system carcinomas, testicular tumor, lung carcinoma, small cell lung carcinoma, non-small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

"Prognosis" as used in this application means the likelihood of recovery from a disease or the prediction of the probable development or outcome of a disease, including but not limited to predicting the length of overall survival, length of disease-free survival, progression-free survival, event-free survival, likelihood of reappearance of cancer in a patient and likelihood of tumor metastasis.

The phrase "overall survival" is well known to one of skill in the art and refers to the fate of the patient after diagnosis, despite the possibility that the cause of death in a patient is not directly due to the effects of the cancer. The phrase "disease-free survival" is well known to one of skill in the art and means living free of the disease being monitored. For example, if GP88 expression is used to diagnose or monitor breast cancer, disease-free survival would mean free from detectable breast cancer. The phrase "likelihood of recovery" is well known to one of skill in the art and refers to the probability of disappearance of tumor or lack of tumor reappearance resulting in the recovery of the patient subsequent to diagnosis of cancer, wherein the probability is determined according to the process of the invention. The phrase "likelihood of reappearance" is well known to one of skill in the art and refers to the probability of tumor reappearance or metastasis in a patient subsequent to diagnosis of cancer, wherein the probability is determined according to the process of the invention. The phrase "event-free survival" is well known to one of skill in the art and means living without the occurrence of a particular group of defined events (for example progression of cancer) after a particular action (e.g., treatment). The phrase "Progression-free survival" is well known to one of skill in the art and refers to the length of time during and after treatment in which a patient is living with a disease that does not get worse, and can be used in a clinical study or trial to help find out how well a treatment is working. The term "metastasis" is well known to one of skill in the art and refers to the growth of a cancerous tumor in an organ or body part, which is not directly connected to the organ of the original cancerous tumor. Metastasis will be understood to include micrometastasis, which is the presence of an undetectable amount of cancerous cells in an organ or body part which is not directly connected to the organ of the original cancerous tumor. Therefore, the present invention contemplates a method of determining the risk of further growth of one or more cancerous tumors in an organ or body part which is not directly connected to the organ of the original cancerous tumor.

As used herein, the phrase "biological sample" encompasses a variety of sample types obtained from a subject and useful in the procedure of the invention. Biological samples may include, but are not limited to, solid tissue samples, liquid tissue samples, biological fluids, aspirates, cells and cell fragments. Specific examples of biological samples include, but are not limited to, solid tissue samples obtained by surgical removal, a pathology specimen, an archived sample, or a biopsy specimen, tissue cultures or cells derived therefrom and the progeny thereof, and sections or smears prepared from any of these sources. Non-limiting examples are samples obtained from breast tissue, lymph nodes, and breast tumors. Biological samples also include any material derived from the body of a vertebrate animal, including, but not limited to, blood, cerebrospinal fluid, serum, plasma, urine, nipple aspirate, fine needle aspirate, tissue lavage such as ductal lavage, saliva, sputum, ascites fluid, liver, kidney, breast, bone, bone marrow, testes, brain, ovary, skin, lung, prostate, thyroid, pancreas, cervix, stomach, intestine, colorectal, brain, bladder, colon, uterine, semen, lymph, vaginal pool, synovial fluid, spinal fluid, head and neck, nasopharynx tumors, amniotic fluid, breast milk, pulmonary sputum or surfactant, urine, fecal matter and other liquid samples of biologic origin, and may refer to either the cells or cell fragments suspended therein, or to the liquid medium and its solutes. All or a portion of the biological sample may have a level of GP88 expression characteristic of one or more disease state(s).

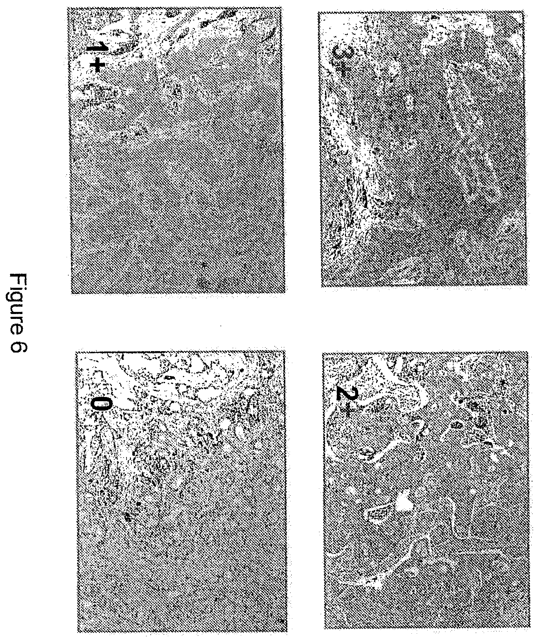

As used herein, a "standard" is a reference that serves as a basis for comparison of other data. A standard may include a biological sample, photographs or photomicrographs of biological samples, or normal ranges (for example, within the range of healthy individuals) derived from an analysis of biological samples. For example, standards may include normal and/or cancer tissue, cancer-free tissue or an archived pathology sample containing GP88 protein expression at various levels for use as positive control, and tumor tissue or other tissue showing no GP88 expression levels as negative control samples, a photograph or photomicrographs, or normal ranges derived from said samples. For example, the photographs in Figure 6 can be considered as standards. Such standards may be used in methods, including but not limited to, for predicting the length of disease-free and overall survival, predicting progression-free survival, predicting the risk of decreased disease-free or overall survival, predicting the likelihood of recovery of a patient suffering from cancer, predicting the likelihood of reappearance of cancer and/or metastasis in an individual having a cancer tumor, predicting the risk of reappearance of cancer, methods for screening a patient suffering from cancer to determine the risk of tumor metastasis, methods for determining the proper course of treatment for a patient suffering from cancer and methods for monitoring the effectiveness of a course of treatment for a patient suffering from cancer.

A "GP88 binding composition" may include any agent, including but not limited to ligands, anti-GP88 antibodies or antigen binding fragments thereof, that is capable of specifically binding to GP88. As used herein, the term "agent that binds to (or capable of binding to) GP88" refers to any molecule that specifically binds to GP88 or polypeptide fragment thereof, including but not limited to, antibodies or antigen-binding fragments thereof, and thereby detects the levels of GP88 expression. Such agents are preferably labeled for detection using methods well known to those of skilled in the art. Examples of labels include, but not limited to, radiolabels, chromophores, fluorophores, enzymes, binding moieties (e.g. biotin) and the like.

Antibodies or antigen-binding fragments thereof, both monoclonal and polyclonal, may be used as GP88 binding composition which binds GP88 protein or a polypeptide fragment thereof. Also contemplated herein as GP88 binding composition any mutants of proteins which specifically bind GP88, whether by deletion (as above exemplified), addition (e.g., addition of a GST domain or a GFP domain), or sequence modification (e.g., site-specific mutagenesis), and the like.

Examples of anti-GP88 antibodies that can be used to measure the concentration of GP88 in a biological fluid sample may be produced from hybridoma cell lines, including, but not limited to, 6B3 hybridoma cell line (ATCC Accession Number PTA-5262), 6B2 hybridoma cell line (ATCC Accession Number PTA-5261), 6C12 hybridoma cell line (ATCC Accession Number PTA-5597), 5B4 hybridoma cell line (ATCC Accession Number PTA5260), 5G6 hybridoma cell line (ATCC Accession Number PTA-5595), 4D1 hybridoma cell line (ATCC Accession Number PTA-5593), 3F8 hybridoma cell line (ATCC Accession Number PTA-5591), 3F5 hybridoma cell line (ATCC Accession Number PTA-5259), 3F4 hybridoma cell line (ATCC Accession Number PTA-5590), 3G2 (ATCC Accession Number PTA-5592), 2A5 hybridoma cell line (ATCC Accession Number PTA-5589), and 4F10 (ATCC Accession Number PTA-8763). All restrictions imposed by the depositor on the availability to the public of the deposited material will be irrevocably removed upon the granting of the patent. In one embodiment of the invention, GP88 monoclonal antibody 6B3, produced by hybridoma cell line (ATCC Number PTA-5262) is used as the primary antibody in immunohistochemistry and sandwich ELISA.

The term antibody herein includes but is not limited to human and non-human polyclonal antibodies, human and non-human monoclonal antibodies (mAbs), chimeric antibodies, anti-idiotypic antibodies (anti-IdAb) and humanized antibodies.

The term antibody is also meant to include both intact molecules as well as fragments thereof such as, for example, Fab, F(ab)₂, Fab', F(ab')₂, Fd, Fd', Fv and scFv, single chain antibodies (natural or recombinant) which are capable of binding to the antigen. The antibody or antigen binding component can be in solution or attached to a support (plate, beads, magnetic beads, etc.,)

The antibodies or fragments of antibodies can be useful immunofluorescence techniques employing a fluorescently labeled antibody (see below) with fluorescent microscopic, flow cytometric, or fluorometric detection. The reaction of antibodies and polypeptides of the present invention may be detected by immunoassay methods well known in the art. The antibodies of the present invention may be employed histologically as in light microscopy, imaging, immunofluorescence or immunoelectron microscopy, for in situ detection of the GP88 protein in tissues samples or biopsies. In situ detection may be accomplished by removing a histological specimen from a patient and applying the appropriately labeled antibody of the present invention.

The biological sample may be treated with a solid phase support or carrier such as nitrocellulose or other solid support capable of immobilizing cells or cell particles or soluble proteins. The support may then be washed followed by treatment with the detectably labeled anti-GP88 antibody. This is followed by wash of the support to remove unbound antibody. The amount of bound label on said support may then be detected by conventional means. By solid phase support is intended any support capable of binding antigen or antibodies such as but not limited to glass, polystyrene polypropylene, nylon, modified cellulose, or polyacrylamide. Alternatively, the antigen may be in solution and the antibody is attached to a support (plate, beads, magnetic beads, etc.,).

The binding activity of a given lot of antibody to the GP88 protein may be determined according to well known methods. Those skilled in the art will be able to determine operative and optimal assay conditions for each determination by employing routine experimentation.

In one embodiment, the invention provides methods for prognosis of one or more disease(s) characterized by, or associated with, a differential (for example, abnormally high and/or abnormally low) expression of GP88. Diseases wherein the level of GP88 expression is high (or elevated) include, but are not limited to, breast cancer, ovarian cancer, prostate cancer, endometrial cancer, etc.; and diseases wherein the level of GP88 expression is low include, but are not limited to, neurodegenerative disorders, comprising: determining the level of GP88 expression in a biological sample obtained from said patient, comparing said level to a standard, and thereby predicting the prognosis associated with said level of GP88 expression.

A preferred embodiment of the invention provides methods for predicting the length of disease-free survival of a patient suffering from one or more disease(s) characterized by, or associated with, a differential (for example, abnormally high and/or abnormally low) expression of GP88, comprising: determining the level of GP88 expression in a biological sample obtained from said patient, comparing said level to standards indicative of healthy individuals or indicative of higher or lower length of disease-free survival, and thereby predicting the length of disease-free survival associated with said level of GP88 expression, wherein elevated levels of GP88 expression is associated with a decreased length of disease-free survival.

A preferred embodiment of the invention provides methods for predicting the length of overall survival of a patient suffering from one or more disease(s) characterized by, or associated with, a differential (for example, abnormally high and/or abnormally low) expression of GP88, comprising: determining the level of GP88 expression in a biological sample obtained from said patient, comparing said level to standards indicative of healthy individuals or indicative of higher or lower length of disease-free survival, and thereby predicting the length of disease-free survival associated with said level of GP88 expression, wherein elevated levels of GP88 expression is associated with a decreased length of overall survival.

A preferred embodiment of the invention provides methods for predicting the likelihood of reappearance of one or more disease(s) characterized by, or associated with, a differential (for example, abnormally high and/or abnormally low) expression of GP88, comprising: determining the level of GP88 expression in a biological sample obtained from said patient, comparing said level to standards indicative of healthy individuals or indicative of higher or lower likelihood of reappearance of said disease, and thereby predicting likelihood of reappearance of said disease associated with said level of GP88 expression, wherein elevated levels of GP88 expression is associated with a decreased likelihood of reappearance of said disease.

A preferred embodiment of the invention provides methods for predicting the length of progression-free survival of a patient suffering from one or more disease(s) characterized by, or associated with, a differential (for example, abnormally high and/or abnormally low) expression of GP88, comprising: determining the levels of GP88 expression in a biological sample obtained from said patient, comparing said levels to standards indicative of healthy individuals or indicative of higher or lower length of disease-free survival, and thereby predicting the length of progression-free survival associated with said level of GP88 expression, wherein elevated levels of GP88 expression is associated with a decreased progression-free survival.

A preferred embodiment of the invention provides methods for predicting the length of disease-free survival of a patient suffering from cancer, comprising: determining the levels of GP88 expression in a biological sample obtained from said patient, comparing said levels to standards indicative of healthy individuals or indicative of higher or lower length of disease-free survival, and thereby predicting the length of disease-free survival associated with said level of GP88 expression, wherein elevated levels of GP88 expression is associated with a decreased length of disease-free survival.

Another embodiment of the invention provides methods for predicting the length of overall survival of a patient suffering from cancer, comprising: determining the levels of GP88 expression in a biological sample obtained from said sample, comparing said levels to standards indicative healthy individuals or indicative of higher or lower length of overall survival, and thereby predicting the length of overall survival associated with said level of GP88 expression, wherein elevated levels of GP88 expression is associated with a decreased length of overall survival.

Another embodiment of the present invention provides methods for predicting the likelihood of recovery of a patient afflicted with cancer, comprising: determining the levels of GP88 expression in a biological sample obtained from said sample, comparing said level to standards indicative of healthy individuals or indicative of higher or lower likelihood of recovery, and thereby predicting the likelihood of recovery associated with said level of GP88 expression, wherein elevated levels of GP88 expression is associated with a decreased likelihood of recovery.

Another embodiment of the present invention provides methods for predicting the progression-free survival of a cancer patient, comprising: determining the levels of GP88 expression in a biological sample obtained from said patient, comparing said level to standards indicative of healthy individuals or indicative of higher or lower progression-free; and thereby predicting the progression-free survival associated with said level of GP88 protein expression, wherein elevated levels of GP88 expression is associated with a decreased progression-free survival of said patient.

Another embodiment of the present invention provides for predicting the risk of decreased disease-free survival of a cancer patient, comprising: determining the levels of GP88 expression in a biological sample obtained from said patient, comparing said level to standards indicative of healthy individuals or indicative of higher or lower risk of decreased disease-free survival; and thereby predicting the risk of decreased disease-free survival associated with said level of GP88 protein expression.

Another embodiment of the present invention provides for predicting the risk of decreased overall survival of a cancer patient, comprising: determining the levels of GP88 expression in a biological sample obtained from said patient, comparing said level to standards indicative of healthy individuals or indicative of higher or lower risk of decreased overall survival; and thereby predicting the risk of decreased overall survival associated with said level of GP88 protein expression.

Another embodiment of the present invention provides methods for predicting the likelihood of recovery of a patient, comprising: determining the level of GP88 expression in a biological sample obtained from said sample, comparing said level to standards indicative of healthy individuals or indicative of higher or lower likelihood of recovery of cancer, and thereby predicting the likelihood of recovery associated with said level of GP88 expression, wherein elevated levels of GP88 expression is associated with a decreased likelihood of recovery of said patient.

Another embodiment of the present invention provides methods for predicting the likelihood of reappearance of cancer in a patient, comprising: determining the level of GP88 expression in a biological sample obtained from said sample, comparing said level to standards indicative of healthy individuals or indicative of higher or lower likelihood of reappearance of cancer, and thereby predicting the likelihood of reappearance of cancer associated with said level of GP88 expression, wherein elevated levels of GP88 expression is associated with a decreased likelihood of reappearance of cancer.

Another embodiment of the present invention provides methods for predicting the likelihood of metastasis of cancer in a patient, comprising: determining the levels of GP88 expression in a biological sample obtained from said patient, comparing said level to standards indicative of healthy individuals or indicative of higher or lower likelihood of metastasis of cancer; and thereby predicting the likelihood of metastasis of cancer associated with said level of GP88 protein expression, wherein elevated levels of GP88 expression is associated with a increased likelihood of metastasis of cancer in a patient.

Another embodiment of the present invention provides methods for predicting the risk of reappearance of cancer in a patient, comprising: determining the levels of GP88 expression in a biological sample obtained from said patient, comparing said level to standards indicative of healthy individuals or indicative of higher or lower risk of reappearance of cancer; and thereby predicting the risk of reappearance of cancer associated with said level of GP88 protein expression, wherein elevated levels of GP88 expression is associated with a increased risk of reappearance of cancer in a patient.

In a further embodiment of the invention, a method is provided for monitoring the effectiveness of a course of treatment for a patient suffering from cancer. This method comprises: a) determining a first level of GP88 expression in a biological sample from said patient prior to said treatment; and (b) subsequently determining a second level of GP88 expression in a biological sample from said patient during said treatment. Comparison of said first level of GP88 expression with said second level of GP88 expression will then indicate the effectiveness of said treatment.

Another embodiment of the invention provides a method for screening a patient afflicted with cancer to determine the risk of tumor reappearance or tumor metastasis. The method comprises determining the levels of GP88 expression in a biological sample obtained from said patient, comparing said level to a standard. A patient found to have elevated levels of GP88, relative to a standard, is classified as being more likely to suffer tumor reappearance or tumor metastasis.

A further preferred embodiment of the invention provides a method for determining the proper course of treatment for a patient suffering from cancer. This method comprises determining the levels of expression of GP88 in a biological sample obtained from a patient. Then, a first group of patients is identified as having low levels of expression of a GP88, which group of patients may require treatment proper for patients having a higher chance of survival or increased time to tumor reappearance or metastasis. The method further comprises identifying a second group of patients as having elevated levels of expression of a GP88, which group of patients may require treatment proper for patients having a lower chance of survival and being more likely to suffer tumor reappearance or metastasis.

In yet another embodiment of the invention, methods are provided for the determination of levels of GP88 expression at an early stage of tumor development. Various stages of tumor development are well known to those of skill in the art, as exemplified in Markman 1997, Basic Cancer Medicine, for example.

The invention is also directed to a method for determining the efficacy of breast conserving surgery (lumpectomy) for treatment of node-negative breast cancer comprising: a) obtaining a biological sample from an individual in need of breast conserving surgery, b) measuring the levels of GP88 expression in said biological sample; and c) comparing said levels to that of a standard to predict the responsiveness of said breast cancer to breast conserving surgery.

Another embodiment of the invention provides methods for prognosis of a patient with cancer, comprising: a) obtaining a biological sample from an individual in need of prognosis; b) determining the level of GP88 expression in a biological sample obtained from said patient; c) scoring said sample for GP88 expression levels; and d) comparing said scoring to that obtained from a control sample (or standard).

The invention is also directed to a method for determining the effect of antiestrogen treatment comprising: a) obtaining a biological sample from an individual in need of antiestrogen treatment, b) measuring the levels of GP88 expression in said biological sample; and c) comparing said levels to a standard to predict the responsiveness to antiestrogen treatment. This method may further comprise a step of determining the proper course of treatment for such patient according to the previously recited methods.

As used herein, "antiestrogen therapy" relates to administration of antiestrogen composition for the purpose of preventing or treating tumor growth. Examples of antiestrogens include estrogen receptor antagonists or SERM (tamoxifen and raloxifene). Other antiestrogen compositions include, aromatase inhibitors (e.g., Arimidex® (anastrozole), Femera®), and estrogen receptor down-regulators (e.g., Faslodex®). The antiestrogenic effects of tamoxifen may be related to its ability to compete with estrogen for binding sites in target tissues. Other antiestrogens, such as aromatase inhibitors, inhibit or reduce the amount of estrogen available.

The invention is also directed to a method for screening compounds comprising: a) obtaining compounds to be screened for their ability to positively or negatively affect GP88 expression; b) contacting a relevant biological sample with said compound; and c) determining the effect of said compound on the levels of GP88 expression in said biological sample. Preferably the effect of said compound may be determined by the binding of an antibody to GP88 to said sample relative to a standard.

### Determining Levels of GP88 Expression

Determination of GP88 expression may be performed by one or more of the methods known to one of ordinary skill in the art. For example, GP88 expression levels may be determined by detection of (a) a GP88 polypeptide, (b) mRNA encoding a GP88 protein, (c) a portion of DNA which constitutes a GP88 gene, or (d) any combination thereof.

For example, levels of GP88 expression can be detected by measuring levels of GP88 protein using GP88 binding compositions. The detection of GP88 protein levels may be carried out using any of the methods known to one of ordinary skill in the art including, but not limited to, chemiluminescence methods, histochemical staining or biochemical detection (i.e., immuno-histochemistry assays), Western Blot analysis, flow cytometry, immuno-precipitation (or the equivalent thereof for non-antibody agents), Plasmon resonance absorbance measurement, and the like. In one embodiment of the invention, the method of detecting GP88 protein levels is an immunoassay (such as an ELISA), which includes the use of at least one antibody. In yet another embodiment of the invention, GP88 staining, in tissue sample for example, formalin-fixed, paraffin-embedded tissue sections can be carried out by immuno-histochemistry using an anti-GP88 antibody, and determining the expression of GP88.

For example, one embodiment of the invention was performed using the OncoStain 88™ IHC kit which uses a primary mouse monoclonal antibody, a secondary anti-mouse IgG antibody, a peroxidase blocker to quench the endogenous peroxidase activity and a chromogenic substrate. Measurement of the polypeptide encoded by a GP88 gene may include measurements of fragments of the polypeptide, wherein the fragments arise from transcriptional or translational variants of the gene; or alternatively, differently sized polypeptides arise as a result of post translational modifications including proteolysis of a larger portion of a GP88 polypeptide.

Detection of levels of mRNA encoding GP88 may also serve as an indicator of GP88 expression. The methods used to detect mRNA levels are well known in the art, and include the detection of hybridization or amplification with the mRNA encoding GP88. This detection may be carried out by analysis of mRNA either in vitro or in situ (e.g., in a tissue sample) using one of the methods known to one of ordinary skill in the art as exemplified in the Current Protocols in Molecular Biology (John Wiley & Sons, 1999); in U.S. Pat. No. 5,882,864; and the like. A GP88 mRNA detected will be any RNA transcript of a GP88 gene, or fragment thereof.

### Classification of Patients

The patients can be classified by comparing the levels of GP88 expression in the biological sample obtained from a patient to a standard. For example, after measuring the GP88 expression level in the sample, the measured level is compared to a standard. This standard is a level of expression of GP88 used to evaluate the level of expression of GP88 in the biological sample of a patient. For example, in one embodiment, when the levels of GP88 expression in the patient sample are higher than that of the standard, the patient sample will be considered to have elevated levels of GP88 expression. Conversely, in another embodiment, when the levels of GP88 expression in the sample are lower than the standard, the sample will be considered to have low levels of GP88 expression.

In another embodiment, patients can be assigned a "score" associated with the GP88 expression in a given biological sample. A sample may be "scored" during the diagnosis or monitoring of breast cancer. Scoring may be determined by the levels of expression of GP88 in a biological sample. In one embodiment, elevated levels of GP88 expression in a biological sample are given a higher score as compared to low levels of GP88 expression, which is given a comparatively lower score. Scoring may also be determined by visual examination of samples by immunohistochemistry. In another embodiment, more quantitative scoring involves determining the two or more parameters, for example (i) intensity of staining and (ii) the proportion of stained ("positive") cells that are sampled. Based on these multiple parameters scores may be assigned that reflect increasing levels of positive staining.

Thus, in one embodiment, a score associated with the levels of GP88 expression in a biological sample obtained from a patient can be compared to the score associated with the levels of GP88 expression in the standard or to cells having no, low or elevated levels of GP88 expression used as controls. Such comparison may provide a basis for better prognosis of the patient. For example, in one embodiment, methods of the invention may score the levels of GP88 expression by using a scale of 0 to 3+, where 0 is negative (no detectable GP88 expression), 1+ and 2+ are associated with a weak and weak to moderate staining, respectively, and 3+ is associated with high intensity staining, in more than 10% of tumor cells; and wherein a lower score indicates a better prognosis of patients.

Prognosis of patients expressing various levels of GP88 can be carried out using single variable or multi-variable analysis. These methods determine the likelihood of a correlation between one or more variables and a given outcome. In one embodiment, the methods will determine the likelihood of a correlation between GP88 expression levels (or GP88 expression levels coupled with another variable) and disease-free or overall survival of cancer patients. Any one of a plurality of methods well known to those of ordinary skill in the art for carrying out these analyses may be used. An example of single variable analysis is the Kaplan-Meir method or the log-rank test. An example of multi-variable analysis is the Cox proportional-hazards regression model. The methods of the invention may further comprise analyzing the levels of GP88 expression in conjunction with additional breast cancer markers. Cox proportional ratio provides a hazard ration or a risk for disease-free and overall survival for patient with varying level of GP88 expression.

Survival analysis using methods of Kaplan and Meier is the recommended statistical technique for use in cancer trials. It is applied by analyzing the distribution of patient survival times following their recruitment to a study. The analysis expresses these in terms of the proportion of patients still alive up to a given time following recruitment. In graphical terms, a plot of the proportion of patients surviving against time has a characteristic decline (often exponential), the steepness of the curve indicating the efficacy of the treatment being investigated. The more shallow the survival curve, the more effective the treatment. Kaplan-Meier analysis can be used to test the statistical significance of differences between the survival curves associated with two different treatments.

In one embodiment, after the levels of expression of GP88 in the sample obtained from a patient have been determined and compared to the standard, the patient is then classified into a group having a certain likelihood of disease-free or overall survival. Then the likelihood of disease-free or overall survival for the patient is assessed based on the likelihood of disease-free or overall survival for patients in that group. For example, the biological sample obtained from a patient may be determined to have elevated levels of GP88 expression relative to the standard. This patient would then be classified into a group of patients having elevated levels of GP88 expression. Since, in accordance with the present invention, it has been discovered that there is a decreased length of disease-free or overall survival for the group of patients expressing elevated levels of GP88, the specific patient afflicted with cancer would be considered to have a decreased length of disease-free or overall survival.

### Kits

The present invention provides a kit to determine the levels of GP88 expression in the biological sample. Such a kit will comprise a reagent for detecting the mRNA encoding GP88, the GP88 polypeptide, or any combination or fragment thereof. The reagent will comprise one or more molecules capable of specifically binding a nucleic acid sequence (DNA or RNA) encoding GP88, or the GP88 polypeptide.

The kit may comprise one or more nucleic acid reagents for the detection of mRNA encoding GP88 (either sense or antisense). The one or more nucleic acid reagents may be used for hybridization and/or amplification of the mRNA encoding GP88. The kit may comprise one or more pairs of primers for amplifying the mRNA encoding GP88. The kit may further comprise samples of total mRNA derived from tissue of various physiological states, such as normal, and metastatically progressive tumor, for example, to be used as controls. The kit may also comprise buffers, nucleotide bases, and other compositions to be used in hybridization and/or amplification reactions. Each solution or composition may be contained in a vial or bottle and all vials held in close confinement in a box for commercial sale. Another embodiment of the present invention encompasses a kit for use in detecting mRNA encoding GP88 in a biological sample comprising oligonucleotide probes effective to bind with elevated affinity to mRNA encoding GP88 in vitro or in situ and containers for each of these probes.

In a further embodiment, the invention encompasses a kit for use in determining the level of GP88 expression in a biological sample comprising one or more agents, such as, for example, one or more antibodies, specific for one or more GP88 polypeptides or fragments. In one particular embodiment, the kit will comprise one or more agents and one or more nucleic acid markers wherein the agents and nucleic acid markers are modified in a fashion appropriate for carrying out immuno-polymerase chain reaction assays.

One preferred embodiment of the invention is directed to a kit for determining the levels of GP88 expression in a mammalian biological sample, wherein said levels of GP88 expression is an indicator of the prognosis of breast cancer, said kit comprising: a) an antibody that specifically binds to GP88 or an antigen binding fragment thereof, b) a reagent useful for detecting the extent of interaction between said antibody and GP88; c) a reagent or solution useful for antigen retrieval; and c) positive and/or negative control samples. Said antibody may be directly linked to an indicator reagent, wherein said indicator reagent is selected from the group consisting of fluorescent, colorimetric, immunoperoxidase and isotopic reagents. Alternatively, the kit may further include a second indicator antibody linked to an indicator reagent, wherein said indicator reagent is selected from the group consisting of fluorescent, calorimetric, immunoperoxidase and isotopic reagents.

In one embodiment, the kit contains at least one primary antibody (e.g., anti-GP88 monoclonal antibody 6B3), at least one labeled secondary antibody (e.g., anti-human GP88 polyclonal antibody labeled with a detection enzyme such as HRP), and at least one substrate (e.g., TMB). Alternatively, the kits can contain radiolabeled secondary antibody in place of the secondary antibody labeled with an enzyme. The kits may also contain disposable supplies for carrying out detection assays (e.g., microtiter plates, pipettes).

It is to be understood that application of the teachings of the present invention to a specific problem or environment will be within the capability of one having ordinary skill in the art in light of the teachings contained herein. The present invention is more fully illustrated by the following non-limiting examples.

### EXAMPLE 1

### GP88 Expression in Invasive Ductal Carcinoma

The current methodology is based on GP88 staining in formalin-fixed, paraffin-embedded human breast lesions investigated with clinical pathological variables. Cytoplasmic GP88 staining was observed in breast carcinoma whereas it was almost always negative in benign breast epithelium. 4-6 micron tissue sections from 203 formalin fixed paraffin embedded biopsies were prepared. GP88 staining was carried out by immuno-histochemistry using anti-human GP88 antibody, and the expression of GP88 was examined in normal tissues, Benign lesions, ductal and lobular carcinomas (Table 1; DCIS: Ductal carcinoma in situ, IDC: Infiltrating carcinoma in situ, LCIS: Lobular carcinoma in situ, ILC: Infiltrating lobular carcinoma). Further, correlation studies of GP88 expression in IDCs with histological grade, proliferation index (Ki67), p53, ER and Her-2 expression were performed.

| **Diagnosis** | **N** | **0** | **1+** | **2+** | **3+** |
|---|---|---|---|---|---|
| **Benign** | **26** | **25 (96%)** | **1 (4%)** | **0** | **0** |
| **DCIS** | **27** | **9 (33%)** | **8 (30%)** | **7 (26%)** | **3 (11%)** |
| **IDC** | **124** | **25 (20%)** | **48 (39%)** | **33 (27%)** | **18 (15%)** |
| **LCIS** | **12** | **11 (92%)** | **1 (8%)** | **0** | **0** |
| **ILC** | **17** | **8 (47%)** | **6 (35%)** | **3 (18%)** | **0** |

### Table 1. Scoring of GP88 Immunostaining

It was observed that GP88 is expressed in both ER+ and ER- tumors, and was predominantly expressed in IDCs with a correlation to histological grade and with Ki67 proliferation index.

### EXAMPLE 2

### Analysis of Levels of GP88 expression in Breast Cancer Tissues of ER-, ER+/LN- and ER+/LN+ Patients

### Study Design

The clinical study was carried out with 389 breast cancer cases (Please see Table 2 for subject characteristics considered in the clinical study), specifically invasive ductal carcinoma tissue samples stored as formalin-fixed, paraffin-embedded blocks and obtained from three tissue repositories. Inclusion criteria are as follows: Year and Age of diagnosis, Tumor Characteristics: Estrogen Receptor (ER), Progesterone Receptor (PR), tumor grade, tumor size, nodal status, Status at last follow-up, Overall survival (OS), Recurrence status, Time until first recurrence, Treatment (ER+, LN0 Tamoxifen). Cases that fit the inclusion criteria were pulled for preparing slides for the study.

### Study Methods

For each case, the histology laboratory from the repository processing site freshly cut 4 - 6 micron tissue sections onto positively charged microscope slides. Slides were examined for section adequacy by the pathologist in charge of pulling the blocks. GP88 expression was determined by staining slides with Oncostain 88™ kit and scored by certified pathologists.

### Materials and Methods

The current method was performed using the OncoStain 88™ IHC kit which uses a primary mouse monoclonal antibody, a secondary anti-mouse IgG antibody, a peroxidase blocker to quench the endogenous peroxidase activity and a chromogenic substrate. The primary mouse antibody binds to human GP88 expressed in the cytoplasm of breast carcinoma cells. This step was followed by the addition of a peroxidase-conjugated secondary antibody that binds to the primary antibody. The specific primary antibody-secondary antibody complex was then visualized with an optimally diluted chromogenic substrate, counterstained and cover slipped. Results were interpreted using a light microscope.

### Results

The staining pattern of the tissue samples was analyzed and categorized (i.e. scored) as shown in Table 3. The levels of GP88 expression was observed for cytoplasmic staining in more than 10% of the tumor cells. Absence of or faint staining observed in less than 10% of the tumor cells was given a score of "0". A weak cytoplasmic staining observed in more than 10% of tumor cells was given a score of "1+", a weak to moderate cytoplasmic staining observed in more than 10% of the tumor cells was given a score of "2+" and a strong cytoplasmic staining observed in more than 10% of the tumor cells was given a score of "3+". Figure 6 shows the reactivity of anti-GP88 with formalin-fixed, paraffin-embedded breast cancer biopsies and the staining pattern, by an indirect immunohistochemical staining method.

The staining patterns resulted above were subjected to further interpretations aid in the assessment of prognosis of breast tumors (See Table 4, below). A score of less than 3+ (i.e. 0, 1+, and 2+) was categorized as GP88 Low Risk Group and were considered to have a decreased or lower risk of reappearance or death; and a score of 3+ was categorized as GP88 Elevated Risk Group and were considered to have a increased or higher risk of reappearance or death.

### Statistical Plan and Analysis

The statistical analysis of results was carried out by a Biostatistician. The statistical analysis plan was based on evaluation of the test performance by its ability to predict disease-free survival and/or overall survival. It was designed to use the log-rank test for identity of pairs of diagnostic groups and the Cox proportional hazards models for quantification of risk in ER+ populations separated by lymph node status (See Table 5 below; OS = Overall Survival, DFS - Disease-free Survival, LNn = Lymph node negative and LNp = Lymph node positive). In addition survival functions for overall and disease-free survival were determined by Kaplan-Meier curves for GP88 3+ group and GP88 < 3+ (GP88 0,1, and 2).

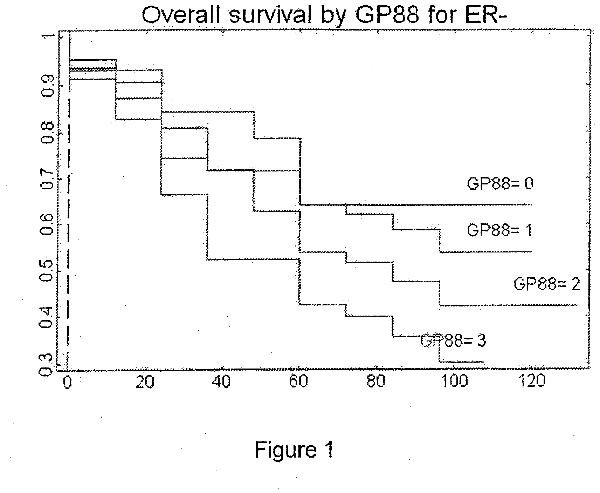

Among ER- patients GP88 showed a statistically significant association with overall mortality (Figure 1). The data showed that patient having ER- cancers that express elevated GP88 level (GP88 3+) have a lower probability of overall survival than ER- cancers with a lower GP88 expression (GP88 0, 1+ or 2+).

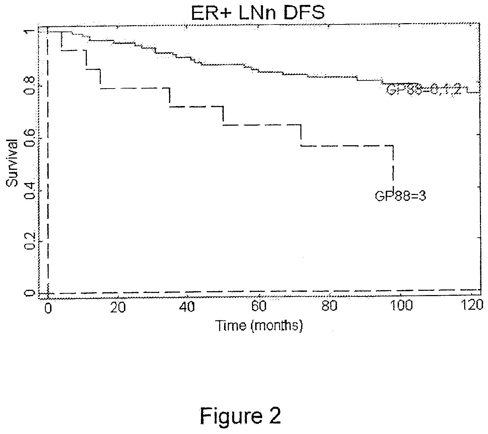

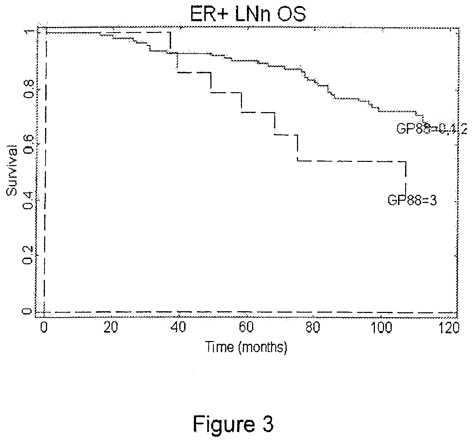

Elevated GP88 expression (3+) is a elevatedly statistically significant indicator of risk of reappearance of tumor in the ER+ / LN- population, and is significantly associated with overall mortality. A elevated level of expression of GP88 (GP88 elevated risk group with a score of 3+ in the cytoplasmic staining) was associated with decreased disease-free survival (Figure 2) and decreased overall survival (Figure 3).

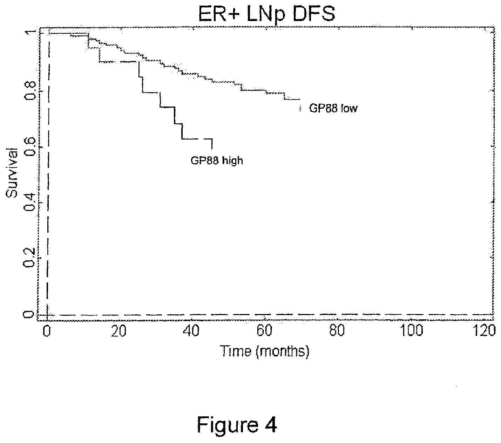

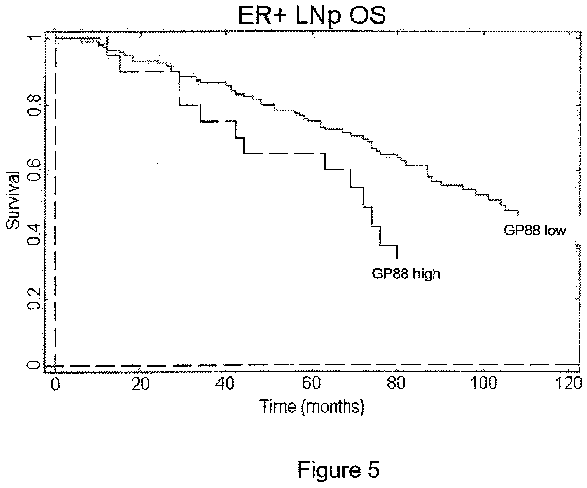

Elevated GP88 expression (3+) was also a elevatedly statistically significant indicator of recurrence risk and overall mortality in the ER+ / LN+ population. A elevated level of expression of GP88 (GP88 elevated risk group with a score of 3+ in the cytoplasmic staining) was associated with decreased disease-free survival (Figure 4) and decreased overall survival (Figure 5).

**Table 2: Subject Characteristics**

| | # of patients |
|---|---|
| Stage 1 | 104 patients |
| Stage 2 | 163 patiens |
| Stage 3 | 112 patients |
| | |

| Tumor Size | |
|---|---|
| 2 cm or less | 174 |
| 2 to 5 cm | 183 |
| > 5 cm | 42 |
| | |

| Grade | |
|---|---|
| I | 18 |
| II | 92 |
| III | 272 |

| ER | |
|---|---|
| Positive | 244 |
| Negative | 142 |
| | |

| PR | |
|---|---|
| Positive | 147 |
| Negative | 168 |

| | |
|---|---|
| Median Age of patients = 56 (26-92) | |

**Table 3: Scoring of Staining Pattern**

| **Staining Pattern** | **GP88 Staining Score** |
|---|---|
| No staining, or faint staining, is observed or cytoplasmic staining is observed in less than 10% of the tumor cells | 0 |
| A weak cytoplasmic staining is observed in more than 10% of tumor cells | 1+ |
| A weak to moderate cytoplasmic staining is observed in more than 10% of the tumor cells | 2+ |
| A strong cytoplasmic staining is observed in more than 10% of the tumor cells | 3+ |

**Table 4: Interpretation of Staining Pattern**

| **Score** | **GP88** | **GP88 Category** | **Interpretation** |
|---|---|---|---|
| 0 | <3+ | GP88 Low Risk Group | Decreased (lower) risk of reappearance or death |
| 1+ | | | |
| 2+ | | | |
| 3+ | 3+ | GP88 Elevated Risk Group | Increased (higher) risk of recurrence or death |

### EXAMPLE 3

### Analysis of Levels of GP88 Expression in biological fluids obtained from Breast Cancer Patients

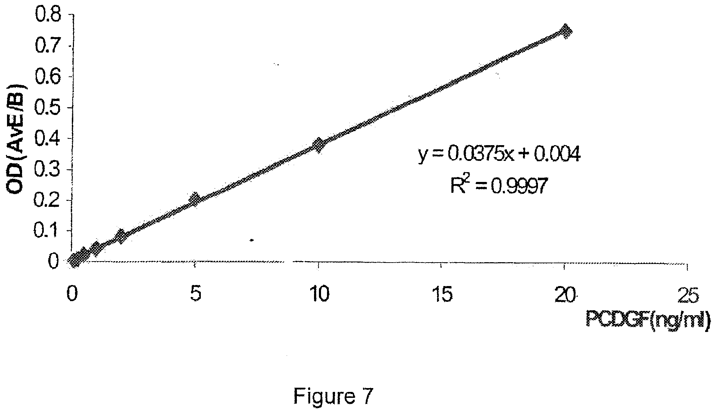

GP88 concentrations in human serum samples were measured in triplicate by enzyme-linked immunoabsorbance assay (ELISA). Standard GP88 samples were prepared from recombinant GP88 diluted in a solution of 30% glycerol and 1% milk-PBS at concentrations of 0, 0.1, 0.25, 0.5, 1, 3, 10, and 20 ng/ml. 100 microliter wells on a microtiter plate were coated with 10 microgram per milliliter of anti-human GP88 monoclonal antibody 6B3 (0.78 mg/ml of 6B3 antibody in phospho buffered saline (PBS)) and incubated overnight at 4°C. The wells were washed with PBS followed by the addition of anti-human PCDGF polyclonal (IgG fraction) to each well at a concentration of 3 micrograms/ml at 37°C. for 1.5 hours. The wells were washed in PBS before the addition of detection antibody (horseradish peroxidase (HRP)-goat-rabbit-IgG) to each well. TMB (substrate) was added and allowed to incubate with the samples for 1 hour. The optical density of the samples was determined using an ELISA spectrometer reader set at a wavelength of 620 nanometers. Plotting the optical density of the standard GP88 samples (y-axis) against the amount of GP88 in each sample (x-axis) generated a standard curve (Figure 7). The GP88 concentration of the unknown samples was determined by measuring the optical density and using the standard curve (Figure 6) to determine the GP88 concentration.

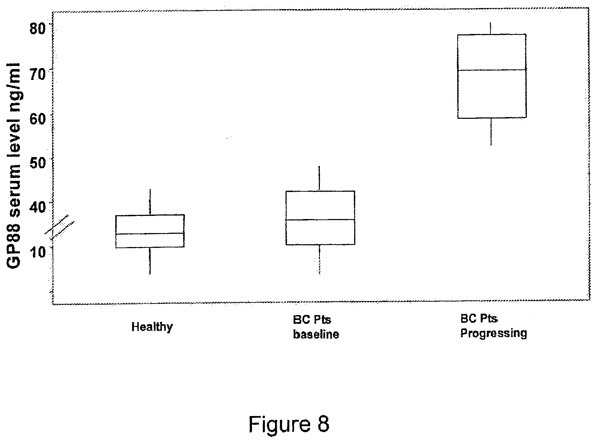

Circulating level of GP88 was measured for patients afflicted with breast cancer patients that have no evidence of disease and that have progressive disease. It was observed (Figure 8) that the level of GP88 in serum of Breast Cancer Patients (BC Pts) with no evidence of disease (baseline) is within the range of healthy individuals. However, BC Pts with progressive disease have significantly higher circulating levels of GP88.

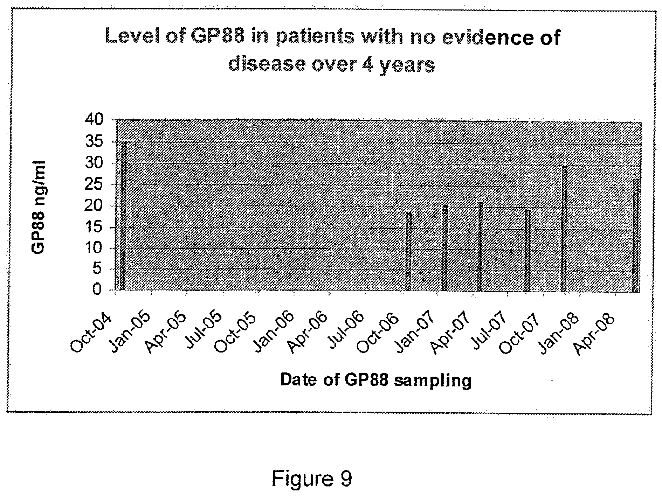

Studies further showed that circulating levels of GP88 expression in breast cancer patients that have no evidence of disease remain low and within the range of healthy individuals. GP88 expression levels were measured (Figure 9) in patients with no evidence of disease, for an extended period of time (i.e. 4 years: Oct. 04 to Apr. 08, as shown in Figure 9). The results showed that patients with early stage disease (Stage 2) that have no evidence of disease maintained stable levels of GP88 that are within the range of healthy individuals.

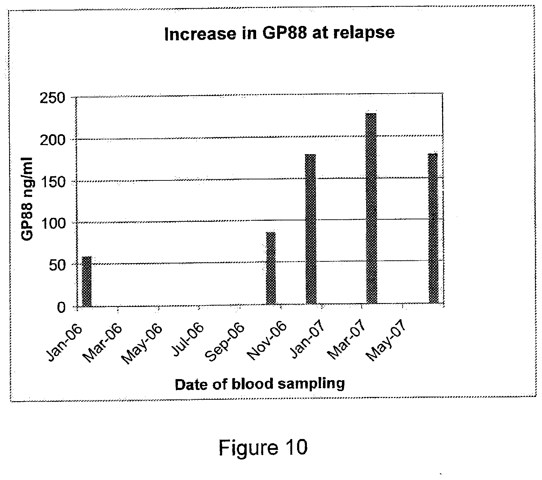

Circulating levels of GP88 was observed for patients that expressed elevated levels of GP88 in the early stage (stage 2) who relapsed to stage 4 disease over time (Approximately, one and half year: Jan 06 to May 07, in the instant case). The data showed (Figure 10) that the patients expressed abnormally elevated levels of GP88 in the late stages.

### Elevated levels of GP88 is associated with loss of survival

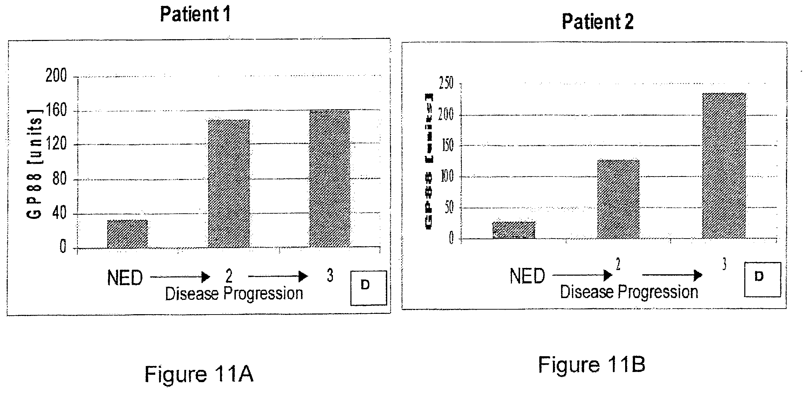

The level of GP88 in the serum of two patients who died of breast cancer was measured using the standard ELISA protocol, and it was observed maintenance of elevated levels of GP88 for several weeks lead to a decrease in survival. The two patients ended the study when they were determined as having no evidence of disease (NED), wherein the GP88 level was low and within the range of healthy individuals (about 20 units - 20 ng/ml). However, over time, levels of GP88 reached to 160 to 200 units (160-200 ng/ml) at which point, patient 1 died within a week (Figure 11A) and patient 2 died with 3 weeks (Figure 11B).

Similar results were observed when the level of GP88 expression was measured in plasma of breast cancer patients, i.e. elevated levels of GP88 was observed when compared to the healthy individuals.

Although the invention has been described with reference to the disclosed embodiments, it should be understood that various modifications can be made without departing from the spirit of the invention. Accordingly, the invention is limited only by the following claims.

### EXAMPLE 4

### Studies to Validate GP88 as a Prognostic Factor in Lung Cancer

As described in the materials and methods of Example 2 above, examination of GP88 expression in tissue microarrays using Oncostain 88™ IHC kit demonstrated GP88 expression in lung cancer tissue from both Squamous Cell Carcinoma and Adenocarcinoma and absence of GP88 expression in normal human tissues including normal human lung tissue (Table 5 below).

**Table 5: GP88 Staining Analysis in Normal and Carcinoma Lung Tissues**

| **Source of tissue for analysis** | **Total # of cases** | **Scoring (% of total)** | | | |
|---|---|---|---|---|---|
| | | **0** | **1+** | **2+** | **3+** |
| **Squamous Cell Carcinoma** | 28 | 8 (29%) | 9 (32%) | 5 (18%) | 6 (21%) |
| **Adenocarcinoma** | 27 | 7 (26%) | 5 (19%) | 7 (26%) | 8 (30%) |
| **Normal Lung** | 5 | 5 (100%) | 0 | 0 | 0 |

### EXAMPLE 5

### Analysis of Levels of GP88 expression in Lung Cancer Tissues

To determine if increasing levels of GP88 in Stage I/II lung cancer tissue correlate with reduced disease-free-survival (DFS) in lung cancer patients, GP88 expression was calculated in 85 cases of resectable stage I/II NSCLC, provided with clinical and outcome data. The tissue was stained using Oncostain 88™ IHC kit and the resulting stained slides were scored for GP88 staining by a Board Certified Pathologist.

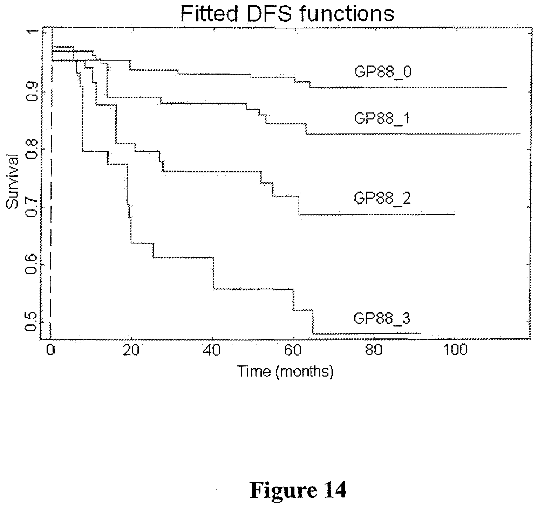

Upon analyzing GP88 scores and survival data using Kaplan-Meier plots, a statistically significant decrease in DFS as GP88 levels increased was observed. This was tested formally by fitting a Cox proportional hazard model using SAS PROC PHREG, treating the GP88 level as an interval-scaled predictor of recurrence. This gave a highly significant association between GP88 and recurrence (P=0.0091). The coefficient of GP88 was 0.676, indicating that each one-unit increase in GP88 corresponded to an approximate doubling of the recurrence hazard (Figure 14).

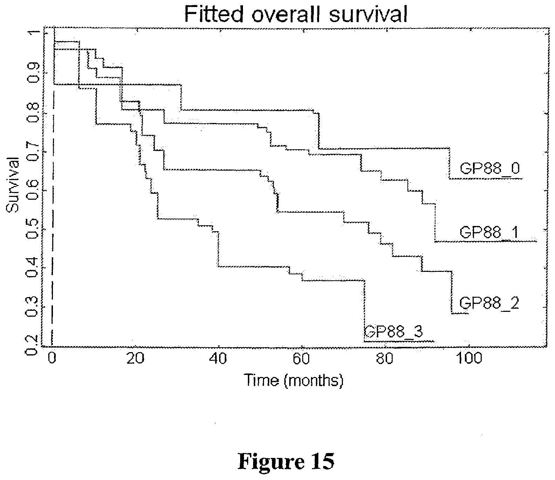

Correlation of GP88 expression with overall survival in the same 85 cases demonstrated a 74% increase in risk of dying for every increase in GP88 score (Figure 15). These data demonstrate that, similar to breast cancer, GP88 expression can be used as a risk predictor of recurrence in, lung cancer, for example, in early stage NSCLC.

### EXAMPLE 6

### Analysis of Levels of GP88 Expression in Biological Fluids Obtained From Lung Cancer Patients

GP88 concentrations in human serum samples obtained from lung cancer patients and healthy non-lung cancer patients were measured by enzyme-linked immunoabsorbance assay (ELISA). Data shown in Table 6 below.

**Table 7. Comparison of GP88 serum levels in lung cancer and healthy non-lung cancer patients.**

| **Type Patient** | **# of Cases** | **GP88 serum levels (ng/ml)** | |
|---|---|---|---|
| | | **Mean** | **Range** |
| **Healthy** | 18 | 28.7 ± 4.25 | 16.6 - 38.2 |
| **Lung Cancer** | 18 | 43.0 ± 10.6 | 28.5 - 73* |

| | | | |
|---|---|---|---|
| *2 patients progressed following initial measurement reaching 120ng/ml | | | |

The data shown in Table 7 indicate that serum GP88 can be used as a predictive indicator in lung cancer. Further, data analysis, using a comparative box and whisker plot, showed a clear difference between the samples obtained from lung cancer and healthy non-lung cancer patients. The Wilcoxon rank sum test confirms this visual impression, yielding an approximate P value of 0.0004.

While the foregoing invention has been described in some detail for purposes of clarity and understanding, it will be appreciated by one skilled in the art from a reading of this disclosure that various changes in form and detail can be made without departing from the true scope of the invention and appended claims. All patents and publications cited herein are entirely incorporated herein by reference.

Aspects of the invention include:
1. A method for prognosis of a patient afflicted with cancer, comprising:
   determining the level of GP88 expression in a biological sample obtained from said patient.
2. A method for prognosis of a patient afflicted with cancer, comprising:
   determining the level of GP88 expression in a biological sample obtained from said patient comprising contacting said biological sample with a GP88 binding composition.
3. A method for prognosis of a patient afflicted with cancer, comprising:
   determining the level of GP88 expression in a biological sample obtained from said patient comprising contacting said biological sample with a GP88 binding composition,
   comparing said level of GP88 expression in said biological sample to a standard, and thereby providing for prognosis associated with said level of GP88 expression.
4. A method of predicting the length of overall survival of a patient with cancer, comprising:
   determining the level of GP88 expression in a biological sample obtained from said patient comprising contacting said biological sample with a GP88 binding composition;
   comparing said level to standards indicative of healthy individuals or indicative of higher or lower overall survival; and thereby predicting the length of overall survival associated with said level of GP88 expression.
5. The method according to aspect 4, wherein an elevated level of GP88 expression is associated with decreased length of overall survival of said patient.
6. A method of predicting the length of disease-free survival of a cancer patient, comprising:
   determining the level of GP88 expression in a biological sample obtained from said patient comprising contacting said biological sample with a GP88 binding composition;
   comparing said level to standards indicative of healthy individuals or indicative of higher or lower disease-free survival; and thereby predicting the length of disease-free survival associated with said level of GP88 protein expression.
7. The method according to aspect 6, wherein an elevated level of GP88 expression is associated with decreased length of disease-free survival of said patient.
8. A method of predicting the progression-free survival of a cancer patient, comprising:
   determining the level of GP88 expression in a biological sample obtained from said patient comprising contacting said biological sample with a GP88 binding composition;
   comparing said level to standards indicative of healthy individuals or indicative of higher or lower progression-free; and thereby predicting the progression-free survival associated with said level of GP88 protein expression.
9. The method according to aspect 8, wherein an elevated level of GP88 expression is associated with decreased progression-free of said patient.
10. A method of predicting the risk of decreased disease-free survival of a cancer patient, comprising:
   determining the level of GP88 expression in a biological sample obtained from said patient comprising contacting said biological sample with a GP88 binding composition;
   comparing said level to standards indicative of healthy individuals or indicative of higher or lower risk of decreased disease-free survival; and thereby predicting the risk of decreased disease-free survival associated with said level of GP88 protein expression.
11. The method according to aspect 10, wherein an elevated level of GP88 expression is associated with the risk of decreased disease-free survival of said patient.
12. A method of predicting the risk of decreased overall survival of a cancer patient, comprising:
   determining the level of GP88 expression in a biological sample obtained from said patient comprising contacting said biological sample with a GP88 binding composition;
   comparing said level to standards indicative of healthy individuals or indicative of higher or lower risk of decreased overall survival; and thereby predicting the risk of decreased overall survival associated with said level of GP88 protein expression.
13. The method according to aspect 12, wherein an elevated level of GP88 expression is associated with the risk of decreased overall survival of said patient.
14. A method of predicting the likelihood of recovery of a patient with cancer, comprising:
   determining the level of GP88 expression in a biological sample obtained from said patient comprising contacting said biological sample with a GP88 binding composition;
   comparing said level to standards indicative of healthy individuals or indicative of higher or lower likelihood of recovery; and thereby predicting the likelihood of recovery associated with said level of GP88 protein expression.
15. The method according to aspect 14, wherein an elevated level of GP88 expression is associated with decreased likelihood of recovery of said patient.
16. A method of predicting the likelihood of reappearance of cancer in a patient, comprising:
   determining the level of GP88 expression in a biological sample obtained from said patient comprising contacting said biological sample with a GP88 binding composition;
   comparing said level to standards indicative of healthy individuals or indicative of higher or lower likelihood of reappearance of cancer; and thereby predicting the likelihood of reappearance of cancer associated with said level of GP88 protein expression.
17. The method according to aspect 16, wherein an elevated level of GP88 expression is associated with decreased likelihood of reappearance of cancer in said patient.
18. A method of predicting the likelihood of metastasis of cancer in a patient, comprising:
   determining the level of GP88 expression in a biological sample obtained from said patient comprising contacting said biological sample with a GP88 binding composition;
   comparing said level to standards indicative of healthy individuals or indicative of higher or lower likelihood of metastasis of cancer; and thereby predicting the likelihood of metastasis of cancer associated with said level of GP88 protein expression.
19. The method according to aspect 18, wherein an elevated level of GP88 expression is associated with decreased likelihood of metastasis of cancer in said patient.
20. A method of predicting the risk of reappearance of cancer in a patient, comprising:
   determining the level of GP88 expression in a biological sample obtained from said patient comprising contacting said biological sample with a GP88 binding composition;
   comparing said level to standards indicative of healthy individuals or indicative of higher or lower risk of reappearance of cancer; and thereby predicting the risk of reappearance of cancer associated with said level of GP88 protein expression.
21. The method according to aspect 20, wherein an elevated level of GP88 expression is associated with increased risk of reappearance of cancer in said patient.
22. A method of monitoring the effectiveness of a course of treatment for a patient suffering from cancer comprising:
   determining a first level of GP88 expression in a biological sample from said patient prior to said treatment; and
   subsequently determining a second level of GP88 expression in a biological sample from said patient during said treatment,
   wherein determining the level of GP88 expression comprises contacting with a GP88 binding composition, and
   comparing said first level of GP88 expression with said second level of GP88 expression to indicate the effectiveness of said treatment.
23. A method of screening a patient afflicted with cancer to determine the risk of tumor metastasis, comprising:
   determining the level of GP88 expression in a biological sample obtained from said patient comprising contacting said biological sample with a GP88 binding composition,
   comparing said level to standards indicative of healthy individuals or indicative of higher or lower risk of tumor metastasis, and thereby predicting the risk of tumor metastasis associated with said level of GP88 protein expression.
24. A method of determining the proper course of treatment for a patient suffering from cancer, comprising:
   determining the level of GP88 expression in a biological sample obtained from said patient comprising contacting said biological sample with a GP88 binding composition, and
   comparing said level to standards indicative of healthy individuals or indicative of higher or lower risk of tumor metastasis.
25. A method according to any one of aspects 1-4, 6, 8, 10, 12, 14, 16, 18, 20, 22, 23 and 24, wherein the cancer is selected from the group consisting of breast, liver, kidney, testes, brain, ovary, skin, lung, prostate, thyroid, pancreas, cervix, colorectal, stomach, intestine, bladder, hematopoietic (lymphoid and myeloid), gastrointestinal (e.g., colon), genitourinary tract (e.g., renal, urothelial cells), uterine, head and neck, nasopharynx, small cell carcinoma, non-small cell carcinoma, Ewing's tumor, squamous cell carcinoma, basal cell carcinoma, adenocarcinoma, melanoma, neuroblastoma, and retinoblastoma.
26. A method according to any one of aspects 1-4, 6, 8, 10, 12, 14, 16, 18, 20, 22, 23 and 24, wherein the biological sample is selected from the group consisting of blood, cerebrospinal fluid, serum, plasma, urine, nipple aspirate, fine needle aspirate, tissue lavage such as ductal lavage, saliva, sputum, ascites fluid, semen, lymph, vaginal pool, synovial fluid, spinal fluid, amniotic fluid, breast milk, pulmonary sputum or surfactant, urine, fecal matter, fluids collected from any of liver, kidney, breast, bone, bone marrow, testes, brain, ovary, skin, lung, prostate, thyroid, pancreas, cervix, stomach, intestine, colorectal, brain, bladder, colon and uterine, head and neck, nasopharynx tumors, and other liquid samples of biologic origin.
27. A method according to any one of aspects 1-4, 6, 8. 10, 12, 14, 16, 18, 20, 22, 23 and 24, wherein the biological is a tissue sample.
28. A method according to any one of aspects 1-4, 6, 8, 10, 12, 14, 16, 18, 20, 22, 23 and 24, wherein said cancer is breast cancer.
29. A method according to any one of aspects 1-4, 6, 8, 10, 12, 14, 16, 18, 20, 22, 23 and 24, wherein said cancer is invasive ductal carcinoma.
30. A method according to any one of aspects 1-4, 6, 8, 10, 12, 14, 16, 18, 20, 22, 23 and 24, wherein said cancer is estrogen receptor negative.
31. A method according to any one of aspects 1-4, 6, 8, 10, 12, 14, 16, 18, 20, 22, 23 and 24, wherein the said cancer is estrogen receptor positive and lymph node negative.
32. A method according to any one of aspects 1-4, 6, 8, 10, 12, 14, 16, 18, 20, 22, 23 and 24, wherein said cancer is estrogen receptor positive and lymph node positive.
33. A method according to any one of aspects 1-4, 6, 8, 10, 12, 14, 16, 18, 20, 22, 23 and 24, wherein said GP88 binding composition is selected from an anti-GP88 antibody or an antigen binding fragment thereof.
34. A method according to aspect 33, wherein said antibody or fragment thereof is polyclonal or monoclonal.
35. A method according to aspect 33, wherein said antigen binding fragment is selected from the group consisting of Fab, F(ab)₂, Fab', F(ab')₂, Fd, Fd', Fv and scFv.
36. A method according to aspect 33, wherein sqaid antibody or fragment thereof is humanized or chimerized antibody.
37. A method according to any one of aspects 1-4, 6, 8, 10, 12, 14, 16, 18, 20, 22, 23 and 24 wherein the cancer is lung cancer.
38. A method according to aspects 1-4, 6, 8, 10, 12, 14, 16, 18, 20, 22, 23 and 24, wherein the cancer is non-small lung carcinoma.
39. A method according to aspect 38, wherein the non-small cell lung carcinoma is selected from a group consisting of squamous cell carcinoma and adenocarcinoma.
40. A method of screening compounds comprising:
   obtaining compounds to be screened for their ability to positively or negatively affect GP88 expression,
   contacting a relevant biological sample with said compound, and
   determining the effect of said compound on the level of GP88 expression in said biological sample.
41. A kit for determining the level of GP88 expression in the biological sample, wherein said kit comprises a container comprising a GP88 binding composition.

## Claims

1. A method of monitoring the effectiveness of a course of treatment for a patient suffering from breast cancer comprising:
determining a first level of GP88 expression in a biological sample from said patient prior to said treatment; and
subsequently determining a second level of GP88 expression in a biological sample from said patient during said treatment,
wherein determining the level of GP88 expression comprises contacting with a GP88 binding composition, and
comparing said first level of GP88 expression with said second level of GP88 expression to indicate the effectiveness of said treatment
wherein an elevated level of GP88 in the second sample compared to the first sample indicates ineffective treatment.

2. The method of Claim 1, wherein the sample is a solid tissue sample.

3. The method of any one of Claims 1 to 2, wherein said GP88 binding composition is selected from an anti-GP88 antibody or an antigen binding fragment thereof.

4. The method of Claim 3, wherein said antibody or fragment thereof is polyclonal or monoclonal.

5. The method of any of Claims 3 to 4, wherein said antigen binding fragment is selected from the group consisting of Fab, F(ab)₂, Fab', F(ab')₂, Fd, Fd', Fv and scFv.

6. The method of any of Claims 3 to 5, wherein said antibody or fragment thereof is humanized or chimerized antibody.

7. The method of claim 2, wherein the solid tissue sample is breast tissue or lymph node.

8. The method of claim 1, wherein the sample is a biological fluid.

9. The method of claim 8, wherein the fluid is serum, blood, plasma, nipple aspirate or breast milk.

10. The method of claim 9, wherein the fluid is serum.
